# FASCICULE DE BREVET EUROPEEN

(11) **EP 2 435 076 B1**
(45) Date de publication et mention de la délivrance du brevet: **14.10.2015**
(21) Numéro de dépôt: 10724364.4
(22) Date de dépôt: 27.05.2010
(51) Int. Cl.: A61K 39/395, A61K 38/17, A61P 35/00, C07K 14/47, C07K 14/435, C12N 15/12, A61K 48/00

(54) **ANTICORPS ANTI-CK POUR L'UTILISATION DANS LE TRAITMENT DU CANCER COLORECTAL**
ANTI-CK8 ANTIKÖRPER ZUR BEHANDLUNG VON KOLOREKTALKARZINOM
ANTI-CK8 ANTIBODIES FOR THE TREATMENT OF COLORECTAL CANCER

(30) Priorité: 27.05.2009 FR 0953510
(43) Date de publication de la demande: 04.04.2012
(73) Titulaire: Université Claude Bernard Lyon 1, 69100 Villeurbanne (FR); Hospices Civils de Lyon, 69002 Lyon (FR); Centre National de la Recherche Scientifique (CNRS), 75016 Paris (FR)
(72) Inventeur: DIAZ, Jean-Jacques, 69200 Venissieux (FR); ROCA-MARTINEZ, Jean-Xavier, 69003 Lyon (FR); SAURIN, Jean-Christophe, 69003 Lyon (FR); PETIT, Serge, 69100 Villeurbanne (FR)
(74) Mandataire: Regimbeau
(86) Numéro de dépôt international: PCT/EP2010/057345
(87) Numéro de publication internationale: WO 2010/136536

(56) Documents cités:
- WO-A-03/057168
- ERLANDSSON ANN ET AL: "Studies of the interactions between the anticytokeratin 8 monoclonal antibody TS1, its antigen and its anti-idiotypic antibody alphaTS1." JOURNAL OF MOLECULAR RECOGNITION : JMR 2003 MAY-JUN, vol. 16, no. 3, mai 2003 (2003-05), pages 157-163, XP002558255 ISSN: 0952-3499
- WASEEM AHMAD ET AL: "Conformational changes in the rod domain of human keratin 8 following heterotypic association with keratin 18 and its implication for filament stability." BIOCHEMISTRY 10 FEB 2004, vol. 43, no. 5, 10 février 2004 (2004-02-10), pages 1283-1295, XP002558256 ISSN: 0006-2960 cité dans la demande
- WEINER GEORGE J: "Monoclonal antibody mechanisms of action in cancer." IMMUNOLOGIC RESEARCH 2007, vol. 39, no. 1-3, 2007, pages 271-278, XP002558257 ISSN: 0257-277X
- ROSSI NORRLUND R ET AL: "Experimental radioimmunotargeting combining nonlabeled, iodine-125-labeled, and anti-idiotypic anticytokeratin monoclonal antibodies: a dosimetric evaluation." CANCER 15 DEC 1997, vol. 80, no. 12 Suppl, 15 décembre 1997 (1997-12-15), pages 2689-2698, XP002558258 ISSN: 0008-543X
- UPASANI OJASWINI S ET AL: "Database on monoclonal antibodies to cytokeratins." ORAL ONCOLOGY MAR 2004, vol. 40, no. 3, mars 2004 (2004-03), pages 236-256, XP002558259 ISSN: 1368-8375

### SEQUENCE LISTING

<110> UNIVERSITE CLAUDE BERNARD LYON 1 HOSPICES CIVILS DE LYON CENTRE NATIONAL DE LA RECHERCHE SCIENTIFIQUE (CNRS) DIAZ, Jean-Jacques ROCA MARTINEZ, Jean-xavier SAURIN, Jean-Christophe PETIT, Serge
<120> Anticorps anti-CK8 pour utilisation comme médicament
<130> D26700
<150> FR 0953510
   <151> 27-05-2009
<160> 2
<170> PatentIn version 3.3
<210> 1
   <211> 483
   <212> PRT
   <213> Homo sapiens
<400> 1
   Met Ser Ile Arg Val Thr Gln Lys Ser Tyr Lys Val Ser Thr Ser Gly
   1 5 10 15
   Pro Arg Ala Phe Ser Ser Arg Ser Tyr Thr Ser Gly Pro Gly Ser Arg
   20 25 30
   Ile Ser Ser Ser Ser Phe Ser Arg Val Gly Ser Ser Asn Phe Arg Gly
   35 40 45
   Gly Leu Gly Gly Gly Tyr Gly Gly Ala Ser Gly Met Gly Gly Ile Thr
   50 55 60
   Ala Val Thr Val Asn Gln Ser Leu Leu Ser Pro Leu Val Leu Glu Val
   65 70 75 80
   Asp Pro Asn Ile Gln Ala Val Arg Thr Gln Glu Lys Glu Gln Ile Lys
   85 90 95
   Thr Leu Asn Asn Lys Phe Ala Ser Phe Ile Asp Lys Val Arg Phe Leu
   100 105 110
   Glu Gln Gln Asn Lys Met Leu Glu Thr Lys Trp Ser Leu Leu Gln Gln
   115 120 125
   Gln Lys Thr Ala Arg Ser Asn Met Asp Asn Met Phe Glu Ser Tyr Ile
   130 135 140
   Asn Asn Leu Arg Arg Gln Leu Glu Thr Leu Gly Gln Glu Lys Leu Lys
   145 150 155 160
   Leu Glu Ala Glu Leu Gly Asn Met Gln Gly Leu Val Glu Asp Phe Lys
   165 170 175
   Asn Lys Tyr Glu Asp Glu Ile Asn Lys Arg Thr Glu Met Glu Asn Glu
   180 185 190
   Phe Val Leu Ile Lys Lys Asp Val Asp Glu Ala Tyr Met Asn Lys Val
   195 200 205
   Glu Leu Glu Ser Arg Leu Glu Gly Leu Thr Asp Glu Ile Asn Phe Leu
   210 215 220
   Arg Gln Leu Tyr Glu Glu Glu Ile Arg Glu Leu Gln Ser Gln Ile Ser
   225 230 235 240
   Asp Thr Ser Val Val Leu Ser Met Asp Asn Ser Arg Ser Leu Asp Met
   245 250 255
   Asp Ser Ile Ile Ala Glu Val Lys Ala Gln Tyr Glu Asp Ile Ala Asn
   260 265 270
   Arg Ser Arg Ala Glu Ala Glu Ser Met Tyr Gln Ile Lys Tyr Glu Glu
   275 280 285
   Leu Gln Ser Leu Ala Gly Lys His Gly Asp Asp Leu Arg Arg Thr Lys
   290 295 300
   Thr Glu Ile Ser Glu Met Asn Arg Asn Ile Ser Arg Leu Gln Ala Glu
   305 310 315 320
   Ile Glu Gly Leu Lys Gly Gln Arg Ala Ser Leu Glu Ala Ala Ile Ala
   325 330 335
   Asp Ala Glu Gln Arg Gly Glu Leu Ala Ile Lys Asp Ala Asn Ala Lys
   340 345 350
   Leu Ser Glu Leu Glu Ala Ala Leu Gln Arg Ala Lys Gln Asp Met Ala
   355 360 365
   Arg Gln Leu Arg Glu Tyr Gln Glu Leu Met Asn Val Lys Leu Ala Leu
   370 375 380
   Asp Ile Glu Ile Ala Thr Tyr Arg Lys Leu Leu Glu Gly Glu Glu Ser
   385 390 395 400
   Arg Leu Glu Ser Gly Met Gln Asn Met Ser Ile His Thr Lys Thr Thr
   405 410 415
   Ser Gly Tyr Ala Gly Gly Leu Ser Ser Ala Tyr Gly Gly Leu Thr Ser
   420 425 430
   Pro Gly Leu Ser Tyr Ser Leu Gly Ser Ser Phe Gly Ser Gly Ala Gly
   435 440 445
   Ser Ser Ser Phe Ser Arg Thr Ser Ser Ser Arg Ala Val Val Val Lys
   450 455 460
   Lys Ile Glu Thr Arg Asp Gly Lys Leu Val Ser Glu Ser Ser Asp Val
   465 470 475 480
   Leu Pro Lys
<210> 2
   <211> 30
   <212> PRT
   <213> Homo sapiens
<400> 2
   Ala Glu Gln Arg Gly Glu Leu Ala Ile Lys Asp Ala Asn Ala Lys Leu
   1 5 10 15
   Ser Glu Leu Glu Ala Ala Leu Gln Arg Ala Lys Gln Asp Met
   20 25 30

## Description

La présente invention concerne des anticorps dirigés contre la cytokératine 8 pour le traitement des cancers colorectaux. L'invention se rapporte aussi à des fragments polypeptidiques dérivés de la cytokératine 8 ainsi qu'à des compositions pharmaceutiques et vaccinales pour la prévention et le traitement des cancers colorectaux.

La cytokératine 8 (CK8) a été décrite comme une protéine appartenant à la classe des filaments intermédiaires, éléments du cytosquelette des cellules épithéliales. La fonction structurale des filaments intermédiaires a fait l'objet de nombreux travaux.

Waseem et al. (Biochemistry, 43 :1283-1295, 2004) ont étudié l'association de la CK8 avec la CK18 et son implication dans la stabilité des filaments de cytokératine. Cette publication décrit notamment différents anticorps se liant aux cytokératines et en particulier à la CK8 dont notamment un anticorps monoclonal M20 se liant spécifiquement à la CK8.

Plus récemment, des protéines de la famille des cytokératines ont été décrites à la surface de cellules de carcinomes. La cytokératine 8 serait ainsi présente à la membrane de différentes cellules cancéreuses. Godfroid et al. (Journal of Cell Science, 99 : 595-607) ont décrit la présence des cytokératines 8, 18 et 19 à la surface de cellules de carcinome du sein mis en culture. Hembrough et al. (Journal of Cell Science, 108 :1071-1082, 1995) ont décrit la présence de CK8 ou d'une protéine « CK8 like » sur la surface des hépatocytes, des cellules HepG2 et des cellules de carcinomes du sein. La cytokératine 8 a également été détectée à la surface de carcinomes d'autres cancers comme le cancer du tractus digestif supérieur (Gires et al., Biochemical and Biophysical Research Communications, 328 :1154-1162, 2005).

Erlandsson et al. (J. of Molecular Recognition, 16 :157-163, 2003) décrit l'anticorps monoclonal TS1 dirigé contre la CK8 humaine et son antiidiotype αTS1 ainsi que leur utilisation en immunothérapie pour le traitement des carcinomes. Selon ce document, l'anticorps TS 1 s'est avéré efficace dans un modèle de souris « nude » d'hepatocarcinome.

La demande internationale WO03/057168 divulgue la présence de nouveaux épitopes à la surface des cellules tumorales de cancer du colon, du sein, des ovaires, du rein, des poumons et des testicules. Ces nouveaux épitopes ne sont pas détectés dans des cellules saines et seraient dérivés des cytokératines 8 et 18 humaines (CK8 et CK18). Selon la demande WO03/057168, ces cytokératines sont clivées dans leur partie N-terminale dans les cellules tumorales et forment un complexe à la surface des cellules exposant de nouveaux épitopes spécifiques des cellules cancéreuses. Cependant, tous ces nouveaux épitopes combinent des séquences de la CK8 et de la CK18.

Le rôle membranaire de la CK8 a été étudié et les résultats obtenus suggèrent que la CK8 est impliquée dans les mécanismes de dégradation de la matrice extracellulaire (MEC). La CK8 aurait un rôle de récepteur du plasminogène à la surface des cellules tumorales. La CK8 participant à la formation d'un complexe protéique permettant le clivage du plasminogène en plasmine. La plasmine étant l'enzyme dégradant les protéines de la matrice extracellulaire.

Doljak et al. (Cancer Letters, 267 :75-84, 2008) ont décrit un anticorps monoclonal se liant au motif VKIALEVEIATY conservé chez différentes cytokératines. Cet anticorps monoclonal se lie notamment à la CK2, à la CK8, à la CK810 et à la CK18 dans des cellules MCF-7 de cancer du sein. Cet anticorps inhibe *in vitro* l'activation du plasminogène sur des cellules MCF-7.

Cependant, malgré l'identification de nombreux antigènes à la surface des cellules tumorales et les progrès réalisés dans la compréhension des mécanismes impliqués, les besoins thérapeutiques restent importants notamment pour traiter les cancers à fort potentiel métastasique, limiter la formation des métastases et limiter la chimiorésistance.

En outre, les métastases sont observées soit d'emblée soit secondairement dans 40 à 60 % des cas de cancers colorectaux. Prédire le risque de métastases est déterminant pour orienter la thérapeutique des patients présentant un cancer colorectal, en particulier pour décider de traiter préventivement le patient avant l'apparition des métastases (traitement adjuvant à la chirurgie). Ainsi, il n'est pas recommandé de réaliser une chimiothérapie adjuvante dans les stades T3N0 et pourtant près de 30 % des patients dans cette catégorie développeront des métastases. Des procédés permettant d'identifier un cancer colorectal ayant un phénotype invasif ou métastatiques présenteraient donc un intérêt déterminant pour un traitement approprié des patients.

Il a maintenant été trouvé dans la présente invention que des anticorps dirigés spécifiquement contre la CK8 et en particulier contre certains fragments dérivés de la CK8 ont un effet inhibiteur sur les capacités invasives et le développement des cellules tumorales de cancer colorectal dans des tests *in vitro* et *in vivo*. En particulier, l'invention concerne l'utilisation de ces anticorps pour le traitement des cancers colorectaux invasifs. Ces anticorps sont donc particulièrement utiles pour la prévention et le traitement des métastases dans le cancer colorectal.

En effet, il a maintenant été mis en évidence que la capacité invasive des cellules tumorales d'adénocarcinome colique induite par la bombésine s'accompagne d'un clivage de la partie C-terminale de la CK-8 humaine. La forme clivée de la CK8 humaine est détectée à la membrane des cellules tumorales. Cette CK8 clivée est ainsi localisée à la surface de structures membranaires de type invadopodes caractéristiques des formes invasives des cellules tumorales.

De façon remarquable, un anticorps monoclonal se liant spécifiquement à la CK8 humaine, dénommé M20, inhibe la capacité invasive de ces cellules tumorales après induction par la bombésine. Cet anticorps reconnaît et se lie de façon spécifique à la partie non clivée de la CK8 humaine présente à la surface des cellules tumorales d'adénocarcinome colique. Au contraire, l'anticorps 1E8, reconnaissant la partie clivée de la CK8 humaine, n'a pas d'effet sur la capacité invasive des cellules tumorales traitées à la bombésine.

En outre, ces résultats *in vitro* obtenus sur des cellules tumorales traitées à la bombésine ont pu être confirmés par des essais *in vivo* sur des souris « nude » après injection en sous-cutané de cellules tumorales ISRECO-1. Chez ces souris, l'anticorps monoclonal M20 inhibe nettement la croissance tumorale.

Alors que de nombreux antigènes, et notamment différentes cytokératines, ont été décrits à la surface des cellules tumorales, il est montré pour la première fois que des anticorps dirigés spécifiquement contre une région de la CK8 humaine ont un effet inhibiteur *in vivo* sur la croissance des cellules tumorales d'adénocarcinome colorectal. En outre, il est montré dans la présente invention que pour obtenir un effet thérapeutique les anticorps se liant à la CK8 humaine doivent se lier à des régions spécifiques de cette CK8 et notamment à la partie non clivée de la molécule. Des fragments polypeptidiques de la CK8 spécifiquement reconnus par l'anticorps M20 ont ainsi été identifiés.

Ces résultats ouvrent des perspectives thérapeutiques entièrement nouvelles pour le traitement des cancers colorectaux et plus particulièrement le traitement des cancers colorectaux invasifs, basés sur des anticorps se liant spécifiquement à certains fragments de la CK8 humaine et en particulier se liant au fragment de la CK8 reconnu par l'anticorps monoclonal M20.

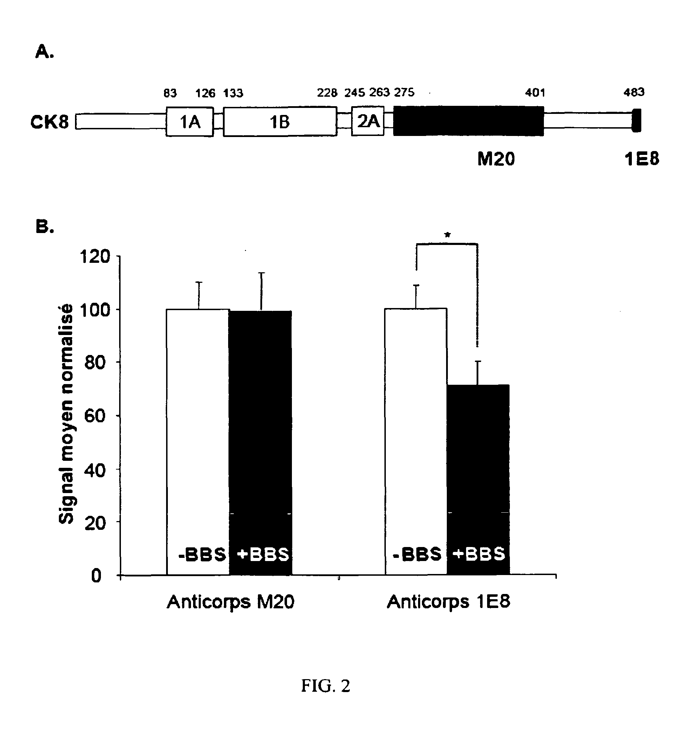

Les résultats obtenus montrent que la CK8 est exposée à la surface des cellules de cancers colorectaux non encore invasifs. La CK8 subit un clivage dans sa partie C-terminale quand la cellule est stimulée par un peptide gastrique qui rend la cellule invasive. Le fragment C-terminal est libéré alors que la partie N-terminale de la CK8 reste exposée à la surface des cellules invasives de cancer colorectal (Figure 2).

La détection du clivage de la CK8 à la surface des cellules de cancer colorectal permet donc d'identifier les cellules invasives ou métastatiques et d'adapter le traitement thérapeutique en conséquence.

### Résumé de l'invention

L'invention se rapporte à des anticorps ou des fragments d'anticorps se liant uniquement au polypeptide dérivé de la CK8 humaine ayant la séquence correspondant aux acides aminés de la position 1 à la position 401 de la SEQ ID No. 1 pour utilisation pour le traitement des cancers colorectaux.

De préférence, les anticorps ou fragments d'anticorps se lient spécifiquement au polypeptide dérivé de la CK8 humaine ayant la séquence correspondant aux acides aminés de la position 275 à la position 401 de la SEQ ID No. 1 et plus préférentiellement les anticorps ou fragments d'anticorps se lient spécifiquement au polypeptide dérivé de la CK8 humaine ayant la séquence correspondant aux acides aminés de la position 338 à la position 367 de la SEQ ID No. 1.

Dans des modes de réalisation, les anticorps ou les fragments d'anticorps se lient spécifiquement à un fragment d'au moins 10 acides aminés du polypeptide dérivé de la CK8 humaine ayant la séquence correspondant aux acides aminés de la position 338 à la position 367 de la SEQ ID No. 1.

Avantageusement, les anticorps ou les fragments d'anticorps selon l'invention sont pour utilisation pour la prévention ou le traitement des cancers colorectaux invasifs et/ou métastatiques.

L'invention se rapporte aussi à des compositions pharmaceutiques comprenant un anticorps selon l'invention et un véhicule pharmaceutique approprié, pour utilisation pour le traitement des cancers colorectaux.

Un autre objet de l'invention est un procédé pour identifier un cancer colorectal ayant un phénotype invasif et/ou métastatique comprenant les étapes suivantes:
- Détection du clivage de la partie C-terminale de la CK8 dans un échantillon préalablement prélevé sur un patient,
- Classifier le cancer colorectal comme invasif et/ou métastatique lorsque la partie C-terminale de la CK8 est clivée.

De préférence, la détection du clivage de la partie C-terminale de la CK8 est effectuée sur un échantillon de cellules de cancer colorectal préalablement prélevé sur un patient.

De préférence, la détection du clivage de la partie C-terminale de la CK8 comprend la détection de la perte totale ou partielle de la partie C-terminale de la CK8 humaine à l'aide d'un anticorps se liant spécifiquement à un polypeptide dérivé de la CK8 humaine ayant la séquence correspondant aux acides aminés de la position 402 à la position 483 de la SEQ ID No. 1.

De préférence, la détection du clivage de la partie C-terminale de la CK8 comprend en outre la détection de la partie N-terminale de la CK8 humaine à l'aide d'un anticorps se liant spécifiquement à un polypeptide dérivé de la CK8 humaine ayant la séquence correspondant aux acides aminés de la position 1 à la position 401 de la SEQ ID No. 1.

Dans les procédés selon l'invention, la détection du clivage de la partie C-terminale de la CK8 dans un échantillon préalablement prélevé sur un patient est effectuée par immuno-histochimie ou immunofluorescence.

Dans des modes de réalisation préférés, la détection du clivage de la partie C-terminale de la CK8 est effectuée dans un échantillon de sang préalablement prélevé sur un patient et dans lequel on détecte dans ledit échantillon de sang la partie C-terminale de la CK8 humaine à l'aide d'un anticorps se liant spécifiquement à un polypeptide dérivé de la CK8 humaine ayant la séquence correspondant aux acides aminés de la position 402 à la position 483 de la SEQ ID No. 1.

L'invention se rapporte aussi à un kit de diagnostic pour identifier un cancer colorectal ayant un phénotype invasif et/ou métastatique comprenant un anticorps se liant spécifiquement à un polypeptide dérivé de la CK8 humaine ayant la séquence correspondant aux acides aminés de la position 402 à la position 483 de la SEQ ID No. 1 et un anticorps se liant spécifiquement à un polypeptide dérivé de la CK8 humaine ayant la séquence correspondant aux acides aminés de la position 1 à la position 401 de la SEQ ID No. 1.

De préférence, les procédés selon l'invention pour identifier un cancer colorectal ayant un phénotype invasif et/ou métastatique sont des procédés *in vitro*.

### Listage des séquences

SEQ ID No. 1 : Séquence en acides aminés de la cytokératine humaine 8 (Genbank P05787)
SEQ ID No. 2: Séquence en acides aminés d'un peptide dérivé de la cytokératine humaine correspondant aux acides aminés A338 à M367.

### Description de l'invention

L'invention se rapporte à l'utilisation à des fins thérapeutiques et à des fins de diagnostic d'anticorps spécifiques des régions N-terminales ou C-terminales de la CK8 humaine. L'invention repose en effet sur l'observation que dans les cellules de cancer colorectaux, l'apparition de cellules invasives et/ou métastatiques s'accompagne d'un clivage de la CK8 humaine exposée à la surface de ces cellules. La partie N-terminale de la CK8 reste exposée à la surface des cellules d'adénocarcinome colique alors que la partie C-terminale de la protéine disparaît partiellement ou totalement de la surface de ces cellules. Des anticorps spécifiques de la partie N-terminale de la CK8 humaine inhibent *in vitro* et *in vivo* la croissance tumorale, le développement de cellules invasives et le développement de métastases. L'apparition de la forme clivée de la CK8 humaine à la surface des cellules d'adénocarcinome colique est un marqueur des phénotypes invasifs et/ou métastatiques.

Les anticorps mis en oeuvre dans la présente invention se lient ou se fixent donc à des fragments sélectionnés de la CK8. De préférence, ces anticorps se lient de façon spécifique à des fragments choisis de la CK8 humaine.

Par « se lier spécifiquement », on entend que ces anticorps se lient uniquement à la CK8 et de préférence uniquement à la CK8 humaine. En particulier, les anticorps ne se lient pas à d'autres antigènes et notamment pas à d'autres cytokératines humaines telles que la CK1, la CK2, la CK10 et la CK18.

Les anticorps selon la présente invention sont de préférence des anticorps monoclonaux spécifiques, notamment d'origines murines, chimériques ou humanisés qui pourront être obtenus selon les méthodes standards bien connues de l'homme du métier.

En général, pour la préparation d'anticorps monoclonaux ou leurs fragments fonctionnels, notamment d'origine murine, on pourra se référer aux techniques qui sont en particulier décrites dans le manuel Antibodies (Harlow and Lane, Antibodies: A Laboratory Manual, Cold Spring Harbor Laboratory, Cold Spring Harbor NY, pp. 726, 1988) ou à la technique de préparation à partir d'hybridomes bien connue de l'homme du métier.

Les anticorps monoclonaux selon l'invention peuvent être obtenus par exemple à partir de cellules d'un animal immunisé avec un fragment de la CK8, en particulier avec le peptide de la SEQ ID No.2, avec un peptide comportant l'épitope reconnu spécifiquement par les anticorps monoclonaux M20 ou avec la partie C-terminale de la CK8 humaine. Lesdits fragments de la CK8 humaine pourront être produits selon les modes opératoires usuels par expression dans un organisme hôte recombinant ou par synthèse peptidique par exemple.

Les anticorps monoclonaux selon l'invention pourront par exemple être purifiés sur une colonne d'affinité sur laquelle a préalablement été immobilisé un fragment de la CK8 humaine, le peptide de la SEQ ID No. 2, un autre peptide comportant l'épitope reconnu spécifiquement par les anticorps monoclonaux M20 ou un fragment dérivé de la partie C-terminale de la CK8 humaine. D'autres techniques de purification bien connues de l'homme du métier pourront être utilisées simultanément ou successivement.

Par anticorps, on entend également les anticorps chimériques ou humanisés.

Par anticorps chimérique, on entend notamment désigner un anticorps qui contient une région variable (chaîne légère et chaîne lourde) naturelle dérivée d'un anticorps d'une espèce donnée en association avec les régions constantes de chaîne légère et chaîne lourde d'un anticorps d'une espèce hétérologue à ladite espèce donnée.

Les anticorps ou leurs fragments de type chimérique selon l'invention peuvent être préparés en utilisant les techniques de recombinaison génétique. Par exemple, l'anticorps chimérique pourra être réalisé en clonant un ADN recombinant comportant un promoteur et une séquence codant pour la région variable d'un anticorps monoclonal non humain, notamment murin, tel que l'anticorps monoclonal M20, et une séquence codant pour la région constante d'anticorps humain. Un anticorps chimérique de l'invention codé par un tel gène recombinant sera par exemple une chimère souris-homme, la spécificité de cet anticorps étant déterminée par la région variable dérivée de l'ADN murin et son isotype déterminé par la région constante dérivée de l'ADN humain. Les méthodes de préparation d'anticorps chimériques sont largement décrites dans la littérature.

Par anticorps humanisé, on entend désigner un anticorps qui contient des régions CDRs dérivées d'un anticorps d'origine non humaine, les autres parties de la molécule d'anticorps étant dérivée d'un (ou de plusieurs) anticorps humains. En outre, certains des résidus des segments du squelette (dénommés FR) peuvent être modifiés pour conserver l'affinité de liaison.

Les anticorps humanisés ou leurs fragments peuvent être préparés par des techniques connues de l'homme du métier. De tels anticorps humanisés sont préférés pour les utilisations thérapeutiques de la présente invention.

L'invention se rapporte aussi à l'utilisation de fragments d'anticorps se liant spécifiquement à un fragment choisi de la CK8 humaine pour le traitement des cancers colorectaux ou pour le diagnostic des formes invasives ou métastatiques du cancer colorectal. Typiquement, ces fragments d'anticorps sont fonctionnels et ils conservent donc leur capacité à se lier ou à se fixer aux fragments de la CK8 humaine et notamment au peptide de la SEQ ID No. 2 dérivé de la CK8 ou à la partie C-terminale de la CK8 humaine.

Par fragment d'un anticorps selon l'invention, on entend désigner en particulier un fragment d'anticorps, tel que des fragments Fv, scFv (sc pour simple chaîne), Fab, F(ab')2, Fab', scFv-Fc ou diabodies, ou tout fragment dont la durée de demi-vie aurait été augmentée par modification chimique. De manière générale, ce fragment est capable d'exercer une activité même partielle de l'anticorps dont il est issu, telle qu'en particulier la capacité à reconnaître et à se lier aux fragments de la CK8. Dans un mode de réalisation avantageux, ce fragment d'anticorps est capable d'inhiber l'invasivité et la croissance des cellules tumorales.

De préférence, lesdits fragments fonctionnels seront constitués ou comprendront une séquence partielle de la chaîne variable lourde ou légère de l'anticorps dont ils sont dérivés, ladite séquence partielle étant suffisante pour retenir la même spécificité de liaison que l'anticorps dont elle est issue et une affinité suffisante, de préférence au moins égale à 1/100, de manière plus préférée à au moins 1/10 de celle de l'anticorps dont elle est issue, vis-à-vis de la CK8 humaine.

Un tel fragment fonctionnel comportera au minimum 5 acides aminés, de préférence 10, 15, 25, 50 et 100 acides aminés consécutifs de la séquence de l'anticorps dont il est issu.

De préférence, ces fragments fonctionnels seront des fragments de type Fv, scFv, Fab, F(ab')2, F(ab'), scFv-Fc ou diabodies, qui possèdent généralement la même spécificité de fixation que l'anticorps dont ils sont issus. Selon la présente invention, des fragments d'anticorps de l'invention peuvent être obtenus à partir des anticorps tels que décrits précédemment par des méthodes telles que la digestion par des enzymes, comme la pepsine ou la papaïne et/ou par clivage des ponts disulfures par réduction chimique. D'une autre manière les fragments d'anticorps compris dans la présente invention peuvent être obtenus par des techniques de recombinaisons génétiques bien connues également de l'homme du métier l'art ou encore par synthèse peptidique.

Un premier objet de l'invention est un anticorps ou fragment d'anticorps pour utilisation pour le traitement des cancers colorectaux, se liant spécifiquement au polypeptide dérivé de la CK8 humaine ayant la séquence correspondant aux acides aminés de la position 1 à la position 367, 380, 393 ou 401 de la SEQ ID No. 1.

L'invention concerne également un anticorps ou fragment d'anticorps pour utilisation pour le traitement des cancers colorectaux, se liant spécifiquement au polypeptide dérivé de la CK8 humaine ayant la séquence correspondant aux acides aminés de la position 275 à la position 367, 380, 393 ou 401 de la SEQ ID No. 1.

L'invention concerne aussi un anticorps ou fragment d'anticorps pour utilisation pour le traitement des cancers colorectaux, se liant spécifiquement au polypeptide dérivé de la CK8 humaine ayant la séquence correspondant aux acides aminés de la position 338 à la position 367, 380, 393 ou 401 de la SEQ ID No. 1.

Plus préférentiellement, l'invention a pour objet un anticorps ou fragment d'anticorps tel que décrit ci-dessus pour utilisation pour la prévention ou le traitement des cancers colorectaux invasifs et/ou métastatiques.

Plus préférentiellement, l'invention a pour objet un anticorps ou fragment d'anticorps tel que décrit ci-dessus pour utilisation pour la prévention ou le traitement des tumeurs métastatiques ou des métastases dans le cancer colorectal.

Un autre objet de l'invention est un procédé pour identifier un cancer colorectal ayant un phénotype invasif et/ou métastatique comprenant les étapes suivantes:
- Détection du clivage de la partie C-terminale de la CK8 dans un échantillon préalablement prélevé sur un patient,
- Classifier le cancer colorectal comme invasif et/ou métastatique lorsque la partie C-terminale de la CK8 est clivée.

En tant que protéines du cytosquelette, les cytokératines ne sont habituellement pas exposées à la surface des cellules saines. Il était connu que dans certains cancers, des épitopes dérivés de cytokératines peuvent être détectés à la surface des cellules tumorales. Il a maintenant été trouvé que la CK8 humaine est exposée à la surface des cellules tumorales dans le cancer colorectal. En outre, la capacité invasive des cellules tumorales d'adénocarcinome colique est caractérisée par le clivage de la partie C-terminale de la CK8 humaine. Seule la partie N-terminale de la CK8 demeure alors à la surface des cellules tumorales. Le site de clivage se situe à proximité de l'extrémité C-terminale du domaine 2B de la cytokératine humaine (domaine correspondant aux positions 275-401 sur la SEQ ID No. 1).

Plus précisément le site de clivage pourrait se situer à la position 393 de la cytokératine humaine de la SEQ ID No. 1.

Les termes « partie N-terminale de la CK8 humaine » réfèrent au polypeptide de la position 1 à la position 401 de la CK8 humaine de la SEQ ID No.1 et de préférence au polypeptide de la position 1 à la position 393 de la CK8 humaine.

Les termes « partie C-terminale de la CK8 humaine » réfèrent au polypeptide de la position 402 à la position 483 de la CK8 humaine de la SEQ ID No. 1 et de préférence au polypeptide de la position 402, 410, 420, 430, 440 ou 450 à la position 483 de la CK8 humaine de la SEQ ID NO.1.

Seuls les anticorps dirigés contre la partie N-terminale de la CK8 humaine ont un effet inhibiteur *in vitro* et *in vivo* sur la croissance tumorale et plus particulièrement les anticorps se liant spécifiquement aux polypeptides dérivés de la CK8 humaine décrits ci-dessus.

Les anticorps dirigés au contraire contre la partie C-terminale n'ont pas d'effet inhibiteur, ces anticorps peuvent être utilisés à des fins de diagnostic pour détecter le clivage de la CK8 humaine et identifier ainsi des cancers colorectaux ayant un phénotype invasif et/ou métastatique.

De préférence, la détection du clivage de la partie C-terminale de la CK8 est effectuée sur un échantillon de cellules de cancer colorectal préalablement prélevé sur un patient.

Par échantillon, on entend dans la présente invention des cellules tumorales prélevées sur un patient tel que par exemple une biopsie de tissu ou d'organe atteint par un cancer colorectal.

Toute méthode appropriée peut être utilisée pour détecter le clivage de la partie C-terminale de la CK8. De préférence, cette détection concerne la détection de la perte d'un fragment C-terminal de la CK8 humaine correspondant au polypeptide de la position 402, 410, 420, 430, 440 ou 450 à la position 483 de la CK8 humaine de la SEQ ID NO.1.

Le clivage peut par exemple être détecté en déterminant le poids moléculaire de la CK8 humaine présente sur les cellules de l'échantillon. La mesure du poids moléculaire permet de détecter la perte de la partie C-terminale de la CK8. Cette opération peut s'effectuer par Western Blot ou par toute autre méthode à l'aide d'un anticorps spécifique de la partie N-terminale de la CK8 humaine.

Alternativement, la détection du clivage de la partie C-terminale de la CK8 humaine peut s'effectuer en mesurant la perte totale ou partielle de la partie C-terminale de la CK8 humaine à la surface des cellules tumorales de l'échantillon. Le clivage et donc la perte de la partie C-terminale de la CK8 peuvent par exemple être détectées à l'aide d'un anticorps spécifique de la partie C-terminale de la CK8 humaine et plus particulièrement d'un anticorps se liant spécifiquement à un polypeptide dérivé de la CK8 correspondant au polypeptide de la position 402, 410, 420, 430, 440 ou 450 à la position 483 de la CK8 humaine de la SEQ ID NO.1.

De préférence, la détection du clivage de la partie C-terminale de la CK8 comprend la détection de la perte totale ou partielle de la partie C-terminale de la CK8 humaine à l'aide d'un anticorps se liant spécifiquement à un polypeptide dérivé de la CK8 humaine ayant la séquence correspondant aux acides aminés de la position 402 à la position 483 de la SEQ ID No. 1.

Avantageusement, la détection du clivage de la CK8 est mesurée à l'aide de deux anticorps se liant respectivement à la partie N-terminale et à la partie C-terminale de la CK8 humaine. Le différentiel du signal obtenu avec les deux anticorps permet de mesurer la perte partielle ou totale de la partie C-terminale de la CK8 humaine à la surface des cellules tumorales de l'échantillon.

De préférence, la détection du clivage de la partie C-terminale de la CK8 comprend en outre la détection de la partie N-terminale de la CK8 humaine à l'aide d'un anticorps se liant spécifiquement à un polypeptide dérivé de la CK8 humaine ayant la séquence correspondant aux acides aminés de la position 1 à la position 393 ou 401 de la SEQ ID No. 1.

Dans les procédés selon l'invention, la détection du clivage de la partie C-terminale de la CK8 dans un échantillon préalablement prélevé sur un patient est par exemple effectuée par immuno-histochimie ou immunofluorescence.
Dans des modes de réalisation préférés, la détection du clivage de la partie C-terminale de la CK8 est effectuée dans un échantillon de sang préalablement prélevé sur un patient et dans lequel on détecte dans ledit échantillon de sang la partie C-terminale de la CK8 humaine à l'aide d'un anticorps se liant spécifiquement à un polypeptide dérivé de la CK8 humaine ayant la séquence correspondant aux acides aminés de la position 402, 410, 420, 430, 440 ou 450 à la position 483 de la CK8 humaine de la SEQ ID NO.1.

L'invention se rapporte aussi à un kit de diagnostic pour identifier un cancer colorectal ayant un phénotype invasif et/ou métastatique comprenant un anticorps se liant spécifiquement à un polypeptide dérivé de la CK8 humaine ayant la séquence correspondant aux acides aminés de la position 402, 410, 420, 430, 440 ou 450 à la position 483 de la SEQ ID No. 1 et un anticorps se liant spécifiquement à un polypeptide dérivé de la CK8 humaine ayant la séquence correspondant aux acides aminés de la position 1 à la position 393 ou 401 de la SEQ ID No. 1.

L'invention a également pour objet un polypeptide choisi dans le groupe consistant en :
a) un polypeptide dérivé de la CK8 humaine ayant la séquence correspondant aux acides aminés de la position 1 à la position 401 de la SEQ ID No. 1,
b) un polypeptide dérivé de la CK8 humaine ayant la séquence correspondant aux acides aminés de la position 275 à la position 401 de la SEQ ID No. 1,
c) un polypeptide dérivé de la CK8 humaine ayant la séquence correspondant aux acides aminés de la position 338 à la position 367 de la SEQ ID No. 1,
d) un fragment d'au moins 10 acides aminés d'un polypeptide dérivé de la CK8 humaine ayant la séquence correspondant aux acides aminés de la position 338 à la position 367 de la SEQ ID No. 1,
e) un polypeptide de la CK8 humaine ayant la séquence correspondant aux acides aminés 402, 410, 420, 430, 440 ou 450 à la position 483 de la SEQ ID No. 1.

L'invention se rapporte donc à des fragments de la CK8 humaine correspondant aux polypeptides ayant la séquence correspondant aux acides aminés de la position 1 à la position 367, 380, 393 ou 401 de la SEQ ID No.1.

L'invention se rapporte aussi à des fragments de la CK8 humaine correspondant aux polypeptides ayant la séquence correspondant aux acides aminés de la position 275 à la position 367, 380, 393 ou 401 de la SEQ ID No.1.

L'invention se rapporte aussi à des fragments de la CK8 humaine correspondant aux polypeptides ayant la séquence correspondant aux acides aminés de la position 338 à la position 367, 380, 393 ou 401 de la SEQ ID No.1.

L'invention se rapporte également à des fragments de ces polypeptides comprenant au moins 5, 10, 15 ou 20 acides aminés.

De préférence, l'invention concerne ces polypeptides pour utilisation comme médicament.

De préférence, l'invention concerne ces polypeptides pour la prévention et/ou le traitement des cancers.

Plus préférentiellement, l'invention concerne ces polypeptides pour la prévention et/ou le traitement des cancers colorectaux et en particulier des cancers colorectaux invasifs ou métastatiques.

La présente invention a donc également pour objet des fragments polypeptidiques ainsi que des peptides dérivés de la CK8 humaine. L'invention se rapporte aussi à des peptides modifiés dérivés de la CK8. Les modifications de peptides sont bien connues de l'homme du métier.

L'invention concerne en particulier le peptide de la SEQ ID No. 2. Ce peptide est reconnu par les anticorps M20, il correspond aux positions 338 à 367 de la CK8 humaine (Genbank P05787 K2C8 Human Keratin). Les anticorps M20 sont spécifiques de la cytokératine humaine 8 et inhibent la croissance et l'invasivité des cellules tumorales dans des tests *in vitro* et *in vivo.*

L'invention concerne également des fragments d'au moins 5, 7, 10, 15, 20 acides aminés du peptide de la SEQ ID No. 2. Ces fragments peptidiques comprennent préférentiellement au moins un épitope du peptide selon la SEQ ID No. 2. Il a été montré dans la présente invention que l'anticorps monoclonal M20 reconnaît spécifiquement le peptide de la SEQ ID No. 2 correspondant aux positions 338 à 367 de la CK8 humaine. De préférence, les fragments peptidiques de la SEQ ID No. 2 comprennent l'épitope (ou déterminant antigénique) reconnu par l'anticorps monoclonal M20 se liant spécifiquement à la CK8 humaine.

Par épitope, on entend une molécule qui peut être reconnue par un paratope (partie variable d'un anticorps ou d'un récepteur membranaire des lymphocytes T : TCR) pour déterminer si elle appartient au domaine du soi ou au domaine du non-soi.

L'anticorps monoclonal M20 commercialisé par Sigma® a été décrit par Van Muijen, G. et al. (Lab. Invest., 57 : 359, 1987). L'épitope antigénique spécifiquement reconnu par l'anticorps M20 pourra être déterminé plus précisément à partir du peptide de la SEQ ID No. 2 selon des techniques bien connues de l'homme du métier. L'homme du métier saura également identifier d'autres épitopes présents dans le peptide de la SEQ ID No. 2 permettant d'éliciter une réponse immune capable d'inhiber la croissance et l'invasivité des cellules tumorales.

De préférence, les fragments du peptide de la SEQ ID No. 2 selon la présente invention comprennent la séquence Lys-Leu (KL) correspondant aux positions 15-16 de la SEQ ID No. 2. Ces acides aminés correspondent aux deux acides aminés K352 et L353 de la CK8 humaine. Il a été montré dans la présente invention que l'épitope de l'anticorps M20 comprend ces séquences.

L'invention a aussi pour objet des peptides présentant au moins 90%, 95%, 98% et préférentiellement au moins 99% d'acides aminés identiques avec le peptide de la SEQ ID No. 2.

Par acides aminés identiques, on entend des acides aminés invariants ou inchangés entre deux séquences. Ces polypeptides peuvent présenter une délétion, une addition ou une substitution d'au moins un acide aminé par rapport au polypeptide de la SEQ ID No. 2.

L'invention a également pour objet des peptides présentant au moins 90%, 95%, 98% et préférentiellement au moins 99% de similarité avec le peptide de la SEQ ID No. 2.

Par similarité, on entend la mesure de la ressemblance entre séquences protéiques. Ces polypeptides peuvent présenter une délétion, une addition ou une substitution d'au moins un acide aminé par rapport au peptide de la SEQ ID No. 2. Le degré de similarité entre deux séquences, quantifié par un score, est basé sur le pourcentage d'identités et/ou de substitutions conservatrices des séquences.

Les méthodes de mesure et d'identification du degré d'identité et du degré de similarité entre polypeptides sont connues de l'homme du métier. On peut employer par exemple Vector NTi 9.1.0, programme d'alignement AlignX (Clustal W algorithm) (Invitrogen INFORMAX, http://www.invitrogen.com). De préférence, on utilise les paramètres par défaut.

L'invention a aussi pour objet des polynucléotides codant pour les polypeptides, les fragments de polypeptides et les peptides dérivés de la CK8 humaine décrits ci-dessus.

Plus préférentiellement, l'invention a pour objet ces polynucléotides pour utilisation comme médicament.

L'invention a également pour objet le peptide de la SEQ ID No. 2 et un polynucléotide codant pour ce peptide.

L'invention se rapporte également à des polynucléotides codant pour des polypeptides et des peptides tels que définis ci-dessus. En raison de la dégénérescence du code génétique, différents polynucléotides peuvent coder pour un polypeptide ou un peptide, tels que définis ci-dessus.

Selon la présente invention, on entend par "polynucléotide" une chaine nucléotidique simple brin ou son complémentaire pouvant être de type ADN ou ARN, ou une chaine nucléotidique double brin pouvant être de type ADNc (complémentaire) ou génomique. De préférence, les polynucléotides de l'invention sont de type ADN, notamment d'ADN double brin. Le terme "polynucléotide" désigne également les polynucléotides modifiés.

Les polynucléotides de la présente invention sont isolés ou purifiés de leur environnement naturel. De préférence, les polynucléotides de la présente invention peuvent être préparés par les techniques classiques de biologie moléculaire telles que décrites par Sambrook et al. (Molecular Cloning : A Labratory Manual, 1989) ou par synthèse chimique.

L'invention a également pour objet une composition pharmaceutique comprennant un anticorps, un polypeptide, un polynucléotide et/ou un peptide selon l'invention.

L'invention concerne des compositions pharmaceutiques comprenant un anticorps, un polypeptide, un peptide ou un polynucléotide tels que définis dans la présente invention et un véhicule pharmaceutique approprié.

Ces compositions peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

L'invention concerne aussi des compositions vaccinales comprenant un polypeptide, un peptide et/ou un polynucléotide, tels que définis dans la présente invention et un adjuvant approprié. En vaccination, les adjuvants sont classiquement définis comme des substances capables de potentialiser ou de moduler la réponse immunitaire contre un ou plusieurs antigènes coadministrés. Les vaccins sont habituellement inoculés par injection, mais ils peuvent l'être par voie orale ou par spray nasal.

L'invention se rapporte à des compositions pharmaceutiques ou vaccinales pour la prévention et/ou le traitement des cancers.

L'invention concerne aussi des méthodes de traitement thérapeutique des cancers colorectaux comprenant l'administration à un individu d'une quantité efficace d'un anticorps se liant spécifiquement aux fragments de la CK8 humaine définis dans la présente invention et notamment au fragment de la SEQ ID No. 2. L'invention concerne aussi des méthodes de traitement thérapeutique des cancers comprenant l'administration à un individu d'une quantité efficace d'un polypeptide ou d'un polynucléotide dérivés de la CK8 tels que décrits dans la présente invention.

L'invention concerne enfin l'utilisation des anticorps, des polypeptides et des polynucléotides tels que définis dans la présente invention pour la fabrication de médicaments pour le traitement des cancers.

Dans un mode de réalisation préféré, la présente invention a pour objet des compositions pour la prévention et/ou le traitement des cancers.

Par « cancer », on entend toutes les formations néoplasiques malignes, quelle qu'en soit la nature histologique. Il existe deux grandes catégories de tumeurs malignes : les carcinomes, d'origine épithéliale, et les sarcomes, d'origine conjonctive. Les tumeurs malignes sont formées de cellules atypiques, envahissantes ou disséminantes, caractérisées généralement par un pouvoir d'accroissement autonome, une délimitation imprécise, une capacité d'envahissement des tissus et vaisseaux voisins et une tendance à disséminer par la production de métastases.

De préférence, les compositions selon l'invention permettent la prévention et/ou le traitement des cancers colorectaux et plus particulièrement des cancers colorectaux invasifs ou métastatiques.

### FIGURES

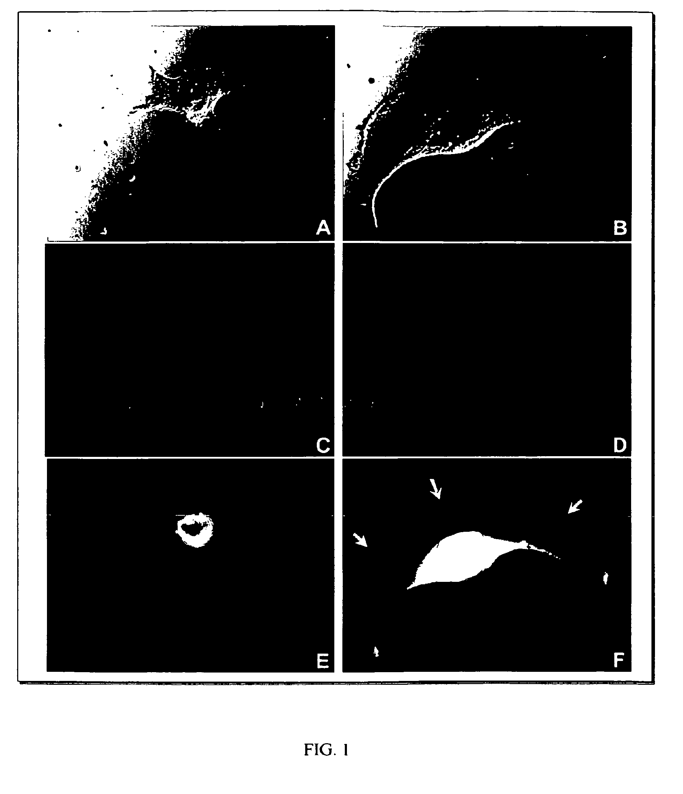

Figure 1 : Analyse par immunofluorescence de la cytokératine 8 dans les cellules modifiées par l'activation bombésine. Des cellules Isreco1 ont été ensemencées sur des lamelles de verre de 12mm ayant au préalablement été recouvertes de Matrigel 1 ng/mL. Elles ont ensuite été traitées pendant 1h dans un milieu contenant de la Bombésine 10 nM. Les lamelles ont été mises en contact avec l'anticorps anti-cytokératine 8 puis avec du DAPI (marqueur nucléaire). A, C et E représentent les images d'une cellule non traitée. B, D et F représentent les images d'une cellule activée par la bombésine. Les photos A et B correspondent au contraste interférentiel différentiel. Les photos C et D visualisent la fluorescence du DAPI. Les photos E et F montrent la fluorescence correspondante à l'anticorps anti-cytokératine 8 (M20). Les flèches jaunes montrent la présence de la CK8 au bord du lamellipode de la cellule.
Figure 2 : Étude par cytométrie de flux de la présence membranaire des épitopes M20 et 1E8 de la cytokératine 8. Trois échantillons de cellules Isreco1 ont été étudiés par cytométrie de flux au cours de quatre expériences différentes (figure 26). Chaque échantillon cellulaire a été réparti en trois fractions marquées respectivement par les anticorps anti-cytokératine 8 M20 et 1E8. la troisième fraction n'étant marquée que par l'anticorps secondaire seul. Une fois le signal résiduel de l'anticorps secondaire soustrait, les signaux des anticorps anti-cytokératine 8 ont été moyennés et normalisés. A. Position des épitopes des anticorps M20 et 1E8 sur la séquence de la cytokératine 8. B. Graphique des moyennes normalisées des signaux de chaque anticorps appliqués au cellules traitées ou non par la bombésine. Significativité : * = p < 0,05.

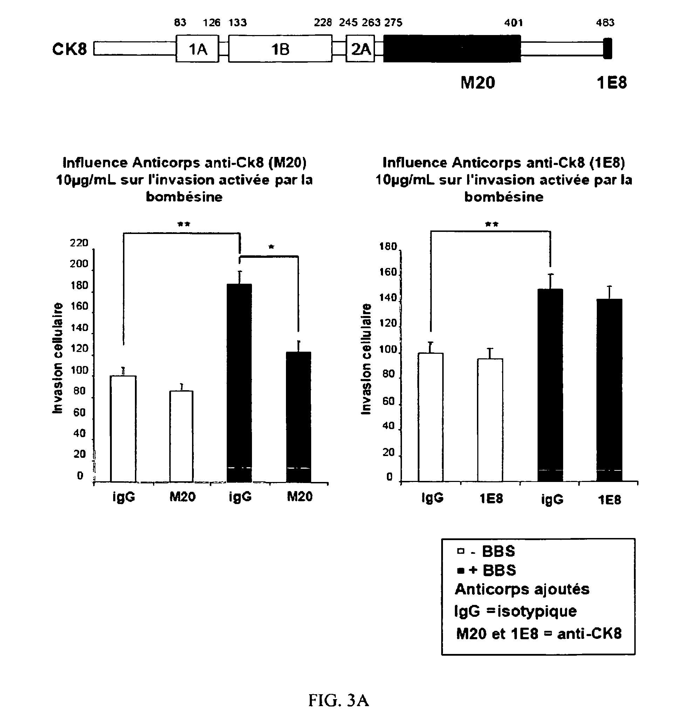

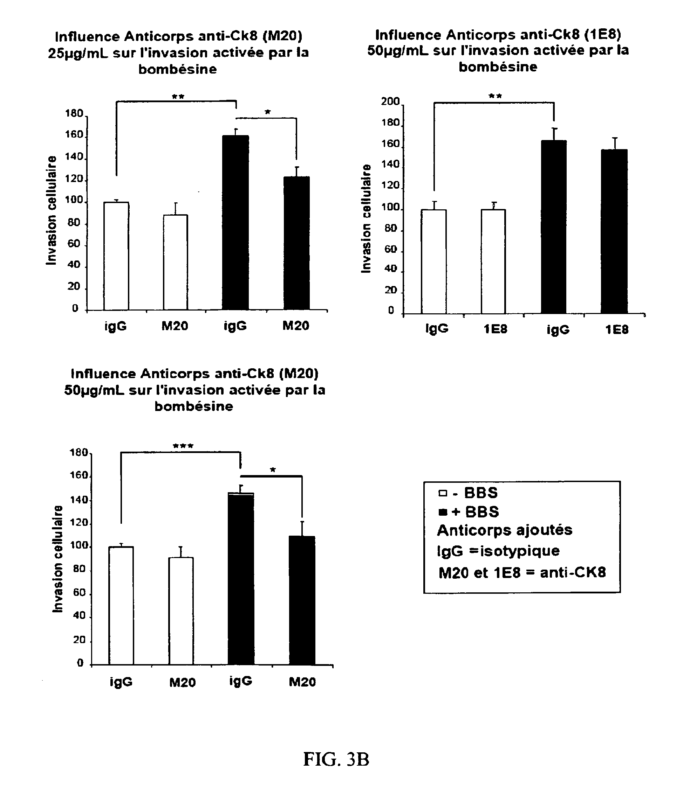

Figure 3 : Étude de l'invasion des cellules Isreco1 induite par l'activation par la bombésine en présence d'anticorps Anti-Cytokératine 8 M20 ou 1E8. Des cellules Isreco1 en suspension ont été incubées avec les anticorps anti-cytokératine 8 (M20 et 1E8) pendant 45 min ou avec un anticorps isotypique correspondant (igG). Ces cellules ont été déposées sur le filtre recouvert de Matrigel d'une chambre de Boyden modifiée. Après 17h d'invasion, les cellules qui ont traversé le filtre sont fixées, colorées puis quantifiées. Les graphiques ci-dessus représentent les moyennes normalisées des absorbances des différents puits de chaque échantillon. Significativité : * = p < 0,05 ; ** = p < 0,005 ; *** = p < 0,001.

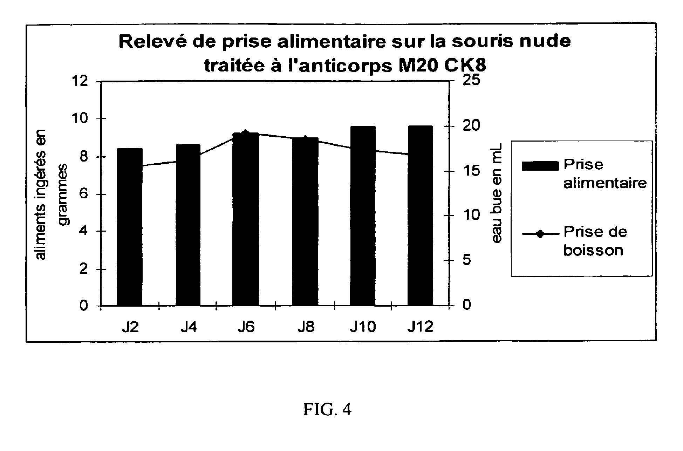

Figure 4 : Relevé de prise alimentaire sur la souris nude traitée à l'anticorps anti-CK8 M20.

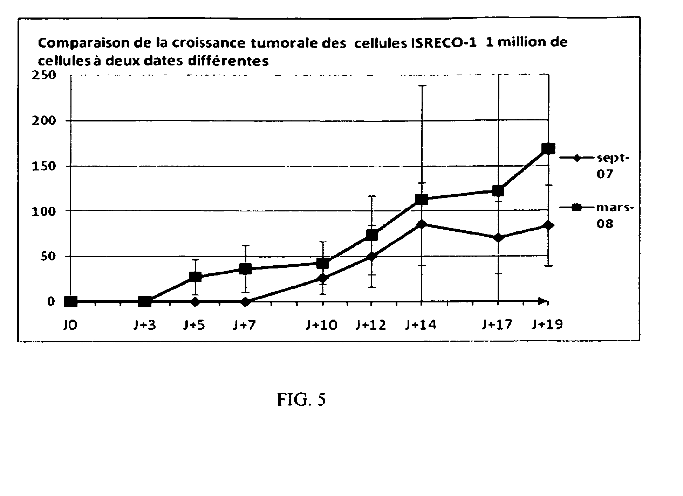

Figure 5 : Comparaison des croissances tumorales des souris traitées avec ISRECO-1.

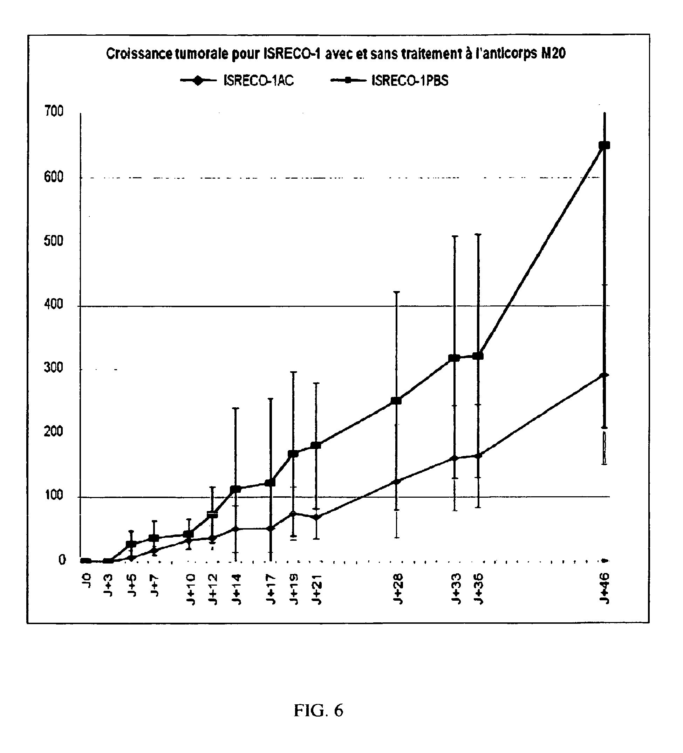

Figure 6 : Comparaison des croissances tumorales des souris traitées et des contrôles.

### EXEMPLES

### Exemple 1 : Cellules, conditions de culture et de traitement à la Bombésine

Voir figure 1.
La lignée cellulaire d'adénocarcinome colique humain Isreco1 a été fournie par le Pr. J-C. Saurin et le Dr. J. Abello (Ancienne Unité Inserm U45, Lyon - actuellemnt Unité INSERM U865). Ces cellules adhérentes ont été cultivées en DMEM (Dubelco's Modified Eagle's Medium, Gibco) contenant 10% de sérum de veau foetal décomplémenté (SVF, Gibco) et 1% de mélange pénicilline - streptomycine (Gibco) et maintenues en atmosphère humide à 37°C et en présence de 5% de CO₂.
Pour le traitement à la bombésine, les cellules sont ensemencées dans le milieu DMEM contenant 0,1% de Bovine Serum Albumin (BSA) et 1% du mélange pénicilline - streptomycine. 48 heures plus tard, le milieu de culture est renouvelé et la bombésine est ajoutée à une concentration finale de 10 nM.

### Exemple 2 : Apparition de d'une forme clivée de la CK8 et caractérisation du clivage

Voir figure 2.
L'analyse comparative de cellules IRESCO-1 non stimulées et stimulées par la Bombésine montre la très forte diminution de l'intensité d'une bande localisée à 53kDa et l'augmentation de l'intensité d'une bande située à 45kDa. Cette observation a été confirmée par une analyse par 2PAGE et par des marquages radioactifs. L'identité de ces deux protéines a été effectuée par spectrométrie de masse : la forme à 53kDa correspond à la forme entière de la CK8, la forme à 53 kDa correspond à une forme clivée. La position du site de clivage a été évaluée par western blot avec des anticorps reconnaissants spécifiquement certains domaines de la CK8. Cette étude nous a permis de définir le site de clivage comme étant situé à la fin du domaine 2B en position 401 et plus précisément aux alentours de la position 393 de la CK8, c'est-à-dire dans le dernier tiers C-terminal de la protéine.

### Exemple 3 : Modulation de l'effet pro-invasif de la BBS par les anticorps anti-CK8

Voir figure 3.
Etant donné que nous avons démontré la présence de CK8 à la surface externe des cellules suite à une stimulation par la bombésine, l'étape suivante a consisté à déterminer si des anticorps dirigés contre la CK8 pouvaient moduler l'acquisition du caractère invasif des cellules Isreco1 stimulées par la BBS. Pour cela, nous avons reproduit le protocole d'invasion cellulaire utilisé pour définir l'activité de la BBS en utilisant des chambres de Boyden modifiées (Saurin et al., Cancer Res, 2002). Ce protocole utilisent une concentration de BBS à 2nM permettant d'obtenir des augmentations de l'invasion de 150 à 200%. Une concentration à 10nM permet d'obtenir des effets plus important dont la quantification est moins précise. Les cellules sont placées dans des chambres de Boyden modifiées. Elles sont soumises ou non à la présence de BBS et placées au dessus d'un film de Matrigel. L'activation de l'invasion induit la mise en place des processus invasifs des cellules Isreco1. Afin de visualiser cet effet et l'influence des anticorps, nous avons mesuré la quantité de cellules ayant franchi le filtre après 17h d'incubation. Les résultats sont basés sur les moyennes obtenues à partir de 7 à 11 puits indépendants par condition. Nous avons comparé l'influence des anticorps anti-CK8 M20 et anti-CK8 1E8 sur l'invasion des cellules Isreco1 au travers d'une membrane recouverte de Matrigel par rapport à celle d'un anticorps isotypique. L'anticorps 1E8 reconnaît la partie qui est clivée suite à la stimulation par la bombésine. Notons que la réalisation de tests d'invasion sans anticorps donne des résultats similaires à l'utilisation d'un anticorps isotypique. Les cellules qui ont envahi la matrice ont été quantifiées et les moyennes des valeurs ont été normalisées en utilisant l'échantillon non traité contenant l'anticorps isotypique comme référence à 100%.

**Tableau 1 : Mesure de la capacité invasive des cellules Isreco1 non traitées ou traitées par la bombésine en présence d'anticorps anti-CK8 M20 ou 1E8**

| | | **Anticorps utilisés** | | | | |
|---|---|---|---|---|---|---|
| | **BBS** | **M20** | | | **1E8** | |
| **Concentrations** | **2nM** | 10 µg/mL | 25 µg/mL | 50 µg/mL | 10 µg/mL | 50µg/mL |
| Isotypique | - | 100 ±8 | 100 ±3 | 100 ±3 | 100 ±5 | 100 ±9 |
| Anti-CK8 | - | 85 ±7 | 88 ±11 | 91 ±10 | 95 ±5 | 100 ±6 |
| Isotypique | + | 187 ±12 | 161 ±7 | 146 ±6 | 150 ±5 | 165 ±6 |
| Anti-CK8 | + | 122 ±11 | 124 ±9 | 101 ±13 | 141 ±5 | 157 ±6 |
| Valeur de p pour l'effet de la BBS | | 0,0047 | 0,0015 | 0,0020 | 0,0015 | 0,0006 |
| Valeur de p pour l'effet de l'anticorps | | 0,030 | 0,039 | 0,017 | 0,548 | 0,586 |

Le tableau 1 présente les résultats pour chaque concentration d'anticorps testée (10, 25 et 50 µg/mL pour l'anti-CK8 M20 et 10 et 50 µg/mL pour l'anti-CK8 1E8) avec les écart-types correspondants. Ainsi, nous avons obtenu une augmentation significative de l'invasion des cellules Isreco1 en présence de bombésine sur l'ensemble des manipulations avec des valeurs comprises entre 146% et 187% d'augmentation (p<0,005). L'ajout de l'anticorps anti-CK8 M20 provoque une diminution significative de la quantité de cellules invasives quelque soit la concentration utilisée (p<0,05). Inversement, l'utilisation de l'anticorps anti-CK8 1E8 n'induit pratiquement pas d'inhibition de l'invasion activée par le traitement pas la BBS (p>0,5). Ce résultat souligne le rôle fonctionnel important des formes de CK8 extramembranaire (clivées ou non) dans l'agressivité des cellules Isreco1 induite par la BBS.

### Exemple 4 : Test de l'innocuité de l'anticorps M20 anti-CK8 sur une souris nude

L'anticorps monoclonal anti-CK8 dénommé M20 est disponible commercialement auprès de la société SIGMA®. Le but de cette étude était d'évaluer si l'anticorps inoculé ne provoque pas d'éventuels dommages physiologiques à la souris nude injectée.

### a) Protocole expérimental

### Espèce animale

Espèce : souris Souche : swiss nude
Sexe : femelle Masse corporelle : 20-25 grammes
Provenance : Charles River Laboratories France
Domaine des oncins-69592 L'Arbresle
Statut sanitaire: Specific Organism Pathogen Free

### Traitement

Quatre injections à la veine caudale de la souris traitée sont effectuées selon le planning suivant :
16/10/2007 (J0)
19/10/2007 (J+5)
23/10/2007 (J+8)
26/10/2007 (J+12)
La dose administrée est de 0,1ml soit 100µg d'anticorps M20.

### b) Résultats

### Morbidité et mortalité :

La souris n'a montré aucun signe de morbidité ni de mortalité.

### Prise alimentaire :

La prise de nourriture et de boisson est de 4,5 grammes de nourriture par jour et 8,6 mL d'eau bue par jour en moyenne. Le relevé de prise alimentaire est représenté à la figure 4.

### Poids de l'animal :

Aucune variation notable de poids n'est à noter durant cette expérience.
Masse corporelle de la souris nude : 23 grammes.
L'injection d'anticorps M20 anti-CK8 à une souris nude n'a pas d'effets sur sa santé ou son comportement.

### Exemple 5 : Etude de l'influence de l'anticorps M20 anti-CK8 sur la croissance des tumeurs ISRECO-1 greffées à des souris nude

Le but de cette étude était d'évaluer l'influence de l'anticorps M20 sur la croissance de tumeurs ISRECO-1 greffées sur des souris nude.

### a) Protocole expérimental :

### Espèce animale :

Espèce : souris Souche : swiss nude
Sexe : femelle Masse corporelle : 20-25 grammes
Provenance : Charles River Laboratories France
   Domaine des Oncins-69592 L'Arbresle
Statut sanitaire: Specific Organism Pathogen Free

**Tableau 2 : Identification des animaux**

| **Groupe « essai » ISRECO-1 AC** | **Groupe « témoin » ISRECO-1 PBS** |
|---|---|
| 1V | 1N |
| 2V | 2N |
| 3V | 3N |
| 4V | 4N |
| VR | NR |
| VN | NV |

### Protocole Opératoire pour les cellules ISRECO-1

### Répartition de 12 souris swiss nude en 2 groupes de 6 souris :

- Groupe « essai »: 6 souris swiss nude destinées à recevoir en intraveineuse des anticorps à partir de J+3 après inoculation des cellules ISRECO-1 en sous cutanée à J0.
- Groupe « témoin »: 6 souris swiss nude destinées à recevoir en intraveineuse du PBS à partir de J+3 après inoculation des cellules ISRECO-1 en sous cutanée à J0.

### La suspension tumorale

La concentration des cellules est de 107 cellules/mL. Injection en sous-cutanée pour chacune des 12 souris (106 cellules ISRECO-1 sous 100µL/souris).

### Les anticorps à tester

A J+3 ; J+6 ; J+10 ; J+13 le groupe « essai » a reçu en intraveineuse 100µg d'anticorps M20 anti-CK8. Aux mêmes dates, le groupe « témoin » a reçu en intraveineuse du PBS stérile aux mêmes volumes que les souris du groupe « essai ».

### Mesure des tumeurs

Les mesures de volumes des tumeurs sont effectuées trois fois par semaine au pied à coulisse numérique selon la formule :
(longueur * (largeur) 2) /2
(Forest et al, Pathology Biology, 52:199-203, 2005).
La dernière mesure s'effectue sur la tumeur extraite après le sacrifice de la souris.

### b) Résultats

### Comparaison de la croissance tumorale des souris nude de septembre 2007 et de mars 2008 inoculées avec ISRECO-1

6 souris ont été inoculées dans chacune des expériences avec 106 cellules ISRECO-1

**Tableau 3: Comparaison des croissances tumorales (en mm3) avec 106 cellules ISRECO-1 (expériences de Septembre 2007 et Mars 2008)**

| **Date** | **J0** | **J+3** | **J+5** | **J+7** | **J+10** | **J+12** | **J+14** | **J+17** | **J+19** |
|---|---|---|---|---|---|---|---|---|---|
| **sept-07** | **0** | **0** | **0** | **0** | **26,59** | **50,56** | **86,03** | **70,52** | **83,87** |
| **mars-08** | **0** | **0** | **27,4** | **36,46** | **43,03** | **73,7** | **113,34** | **122,3** | **168,5** |
| écart type sept 07 | 0 | 0 | 0 | 0 | 18,07 | 34,11 | 45,69 | 39,5 | 44,7 |
| écart type mars 08 | 0 | 0 | 19,6 | 26,5 | 23,7 | 43,8 | 126,3 | 131,8 | 128,8 |

La comparaison des croissances tumorales des souris traitées avec ISRECO-1 est présentée à la figure 5.
La croissance tumorale pour ISRECO-1 injecté en mars 2008 commence à J+3 alors qu'en septembre 2007 celle-ci commence à J+7. A J+19, le volume moyen des tumeurs en mars 2008 est 2 fois plus important qu'en septembre 2007.
Souris inoculées avec les cellules ISRECO-1 : Comparaison de la croissance tumorale (volume en mm3) des souris traitées avec l'anticorps M20 et des souris non traitées (contrôles PBS).
Après inoculation de 106 cellules, un groupe de 6 souris a été traité avec l'anticorps M20 et un groupe contrôle de 6 souris a reçu le diluant seul (tampon PBS stérile, 0,14M pH neutre à J+3 ; J+6 ; J+10 ; J+13).

**Tableau 4 : Comparaison de la croissance tumorale (volume moyen en mm3) des souris traitées avec l'anticorps M20 et des souris non traitées (contrôles PBS).**

| **Date** | **J0** | **J+3** | **J+5** | **J+7** | **J+10** | **J+12** | **J+14** | **J+17** | **J+19** | **J+21** | **J+28** | **J+33** | **J+35** | **J+46** |
|---|---|---|---|---|---|---|---|---|---|---|---|---|---|---|
| **ISRECO-1 AC** | 0 | 0 | 6,3 | 16,9 | 33,2 | 36,9 | 50,6 | 51 | 74,6 | 68,9 | 124,25 | 161,24 | 164,27 | 291,12 |
| **ISRECO-1 PBS** | 0 | 0 | 27,4 | 36,5 | 42,5 | 73,7 | 113 | 122,3 | 168,5 | 181,1 | 250,91 | 318,58 | 320,65 | 649,87 |
| **écartype AC** | 0 | 0 | 9,892 | 7,48 | 13,09 | 18,78 | 36,7 | 36,96 | 41,36 | 33,77 | 88,54 | 81,55 | 80,03 | 140,5 |
| **écartype PBS** | 0 | 0 | 19,6 | 26,5 | 23,75 | 43,82 | 126 | 131,9 | 128,8 | 98,63 | 170,75 | 189,49 | 190,3 | 441,98 |

La croissance tumorale des souris traitées avec ISRECO-1 est inhibée dès la première injection de l'anticorps à J+3. A J+46, la taille moyenne est de 291,12 mm3 pour le groupe ISRECO-1 AC soit 2.23 fois inférieure au volume moyen tumoral pour les souris non traitées (649,87 mm3). La comparaison des croissances tumorales des souris traitées et des contrôles est représentée à la figure 6.

Dans les tableaux 5 et 6 sont reportées les différentes valeurs trouvées pour chacune des souris.

**Tableau 5: Croissance tumorale des souris ayant reçu 106 cellules ISRECO-1 traitées avec de l'anticorps M20 (volume en mm3)**

| **Date** | **J0** | **J+3** | | **J+5** | **J+7** | **J+10** | **J+12** | | **J+14** | | **J+17** |
|---|---|---|---|---|---|---|---|---|---|---|---|
| 1V | 0 | 0 | | 0 | 12,2 | 15,56 | 18,92 | | 20,6 | | 21,02 |
| 2V | 0 | 0 | | 0 | 12,2 | 34,91 | 33,69 | | 39,8 | | 43,42 |
| 3V | 0 | 0 | | 0 | 12,2 | 35,91 | 24,99 | | 27,5 | | 25,23 |
| 4V | 0 | 0 | | 16,38 | 16,8 | 46,21 | 60,86 | | 111 | | 110 |
| VR | 0 | 0 | | 21,43 | 31,5 | 46,75 | 59,74 | | 80,6 | | 82,5 |
| VN | 0 | 0 | | 0 | 16,4 | 19,7 | 23,06 | | 24,5 | | 23,89 |
| MOYENNE | 0 | 0 | | 6,302 | 16,9 | 33,17 | 36,88 | | 50,6 | | 51,01 |
| écartype | 0 | 0 | | 9,892 | 7,48 | 13,09 | 18,78 | | 36,7 | | 36,96 |
| | | | | | | | | | | | |

| **Date** | **J+19** | | **J+21** | | **J+28** | **J+33** | | **J+35** | | **J+46** | |
|---|---|---|---|---|---|---|---|---|---|---|---|
| 1V | 33,05 | | 31,18 | | 71,41 | 89,73 | | 94,64 | | 53,23 | |
| 2V | 69,86 | | 60,08 | | 78,73 | 130,62 | | 138,5 | | 443,6 | |
| 3V | 44,87 | | 51,16 | | 102,04 | 192,2 | | 194,12 | | 392,67 | |
| 4V | 115,2 | | 101,3 | | 112,5 | 168,68 | | 170,67 | | 253,9 | |
| VR | 135 | | 119 | | 302,09 | 303 | | 303 | | 360,85 | |
| VN | 49,61 | | 50,62 | | 78,73 | 83,19 | | 84,7 | | 242,5 | |
| MOYENNE | 74,6 | | 68,88 | | 124,25 | 161,237 | | 164,27 | | 291,13 | |
| écartype | 41,36 | | 33,78 | | 88,538 | 81,551 | | 80,027 | | 140,55 | |

**Tableau 6: Croissance tumorale des souris ayant reçu 106 cellules ISRECO-1 traitées avec du PBS stérile (volume en mm3)**

| **Date** | **J0** | **J+3** | **J+5** | **J+7** | **J+10** | **J+12** | **J+14** | **J+17** |
|---|---|---|---|---|---|---|---|---|
| 1N | 0 | 0 | 0 | 12,2 | 23,7 | 35,38 | 32 | 39,69 |
| 2N | 0 | 0 | 56,25 | 56,3 | 67,97 | 90,04 | 72,6 | 76,47 |
| 3N | 0 | 0 | 31,18 | 79,5 | 71,94 | 147,9 | 366 | 385,3 |
| 4N | 0 | 0 | 12,19 | 12,2 | 12,19 | 25,6 | 41,2 | 38,66 |
| NR | 0 | 0 | 37,26 | 29,5 | 36,82 | 67,5 | 106 | 113,5 |
| NV | 0 | 0 | 27,74 | 29,2 | 42,59 | 75,71 | 62,5 | 80,06 |
| MOYENNE | 0 | 0 | 27,44 | 36,5 | 42,54 | 73,7 | 113 | 122,3 |
| écartype | 0 | 0 | 19,63 | 26,5 | 23,75 | 43,82 | 126 | 131,9 |
| | | | | | | | | |

| **Date** | **J+19** | **J+21** | | **J+28** | **J+33** | **J+35** | | **J+46** |
|---|---|---|---|---|---|---|---|---|
| 1N | 37,26 | 58,82 | | 58,82 | 74,44 | 76 | | 109,8 |
| 2N | 115,3 | 147,8 | | 167,13 | 261 | 261 | | 800 |
| 3N | 373,5 | 320,6 | | 451,2 | 599,04 | 600,3 | | 1310 |
| 4N | 56,7 | 104,4 | | 115,75 | 167,4 | 167,4 | | 283,27 |
| NR | 259,9 | 265,7 | | 459,42 | 416 | 418 | | 907,5 |
| NV | 168,3 | 189 | | 253,12 | 393,58 | 401,2 | | 488,67 |
| MOYENNE | 168,5 | 181,1 | | 250,91 | 318,577 | 320,65 | | 649,87 |
| écartype | 128,8 | 98,62 | | 170,75 | 189,499 | 190,3 | | 441,98 |

La dernière mesure a été réalisée après l'extraction de la tumeur. L'épiderme de la souris a été retiré et le volume de la tumeur a été calculé comme décrit précédemment.

Dans le tableau 7 sont reportés pour chaque souris les délais (en jours) pour atteindre différents volumes de tumeurs. (50, 100, 150 et 200 mm3).

| Souris | Délai (en jour) pour 50 mm³ | Délai (en jour) pour 100 mm³ | Délai (en jour) pour 150 mm³ | Délai (en jour) pour 180 mm³ | Délai (en jour) pour 200 mm³ |
|---|---|---|---|---|---|
| Traitées 1V | 26 | | | | |
| Traitées 2V | 18 | 30 | 37 | 41 | 44 |
| Traitées 3V | 20 | 28 | 30 | 32 | 36 |
| Traitées 4V | 11 | 13 | 30 | 42 | 44 |
| Traitées VR | 11 | 18 | 24 | 26 | 26 |
| Traitées VN | 21 | 38 | 41 | 44 | 44 |
| **Moyenne** | **17,8** | **25,4** | **32,4** | **37** | **38,8** |
| écartype | 5,9 | 9,9 | 6,6 | 7,7 | 7,9 |

**Tableau 7: Délais pour atteindre 50, 100, 150 et 200 mm cube de volume tumoral par souris**

| | | | | | |
|---|---|---|---|---|---|
| Contrôle 1N | 20 | 45 | | | |
| Contrôle 2N | 4 | 18 | 22 | 32 | 32 |
| contrôle 3N | 6 | 11 | 13 | 13 | 13 |
| contrôle 4N | 18 | 20 | 32 | 37 | 37 |
| contrôle NR | 11 | 13 | 18 | 18 | 18 |
| contrôle NV | 11 | 13 | 18 | 21 | 24 |
| **Moyenne** | **11,6** | **20** | **20,6** | **24,2** | **24,8** |
| écartype | 6,6 | 10,9 | 7,1 | 9,9 | 9,8 |

Les tumeurs ISRECO-1 traitées avec de l'anticorps M20 atteignent 50 mm3 en 18 jours et évoluent jusqu'à 200 mm3 en 38,8 jours. En revanche, les tumeurs ISRECO-1 non traitées ont un retard de croissance par rapport aux traitées de 5 jours pour l'atteinte de 100 mm3 puis 14 jours pour l'atteinte à 200 mm3.
Pour passer de 100 à 200 mm3, les tumeurs des souris traitées ont un temps de doublement de 13,4 jours alors que pour les souris contrôles le temps de doublement est beaucoup plus rapide (4,8 jours).
L'anticorps M20 anti-CK8 inhibe la croissance tumorale chez des souris inoculées avec les cellules ISRECO-1.

### REFERENCES BIBLIOGRAPHIQUES

Doljak et al., Cancer Letters, 267 :75-84, 2008
Erlandsson et al. (J. of Molecular Recognition, 16 :157-163, 2003)
Forest et al., Pathology Biology, 52:199-203, 2005
Gires et al., Biochemical and Biophysical Research Communications, 328:1154-1162, 2005
Godfroid et al., Journal of Cell Science, 99:595-607
Hembrough et al., Journal of Cell Science, 108:1071-1082, 1995
Van Muijen, G. et al., Lab. Invest., 57:359, 1987
Waseem et al., Biochemistry, 43 :1283-1295, 2004

### REFERENCES BREVET

WO03/057168

## Revendications

1. Anticorps ou fragment d'anticorps, se liant uniquement au polypeptide ayant la séquence des acides aminés de la position 1 à la position 401 de la SEQ ID No. 1 de la CK8 humaine pour utilisation pour le traitement des cancers colorectaux.

2. Anticorps ou fragment d'anticorps selon la revendication 1, se liant uniquement au polypeptide ayant la séquence des acides aminés de la position 275 à la position 401 de la SEQ ID No. 1 de la CK8 humaine.

3. Anticorps ou fragment d'anticorps selon l'une des revendications précédentes, se liant uniquement au polypeptide ayant la séquence des acides aminés de la position 338 à la position 367 de la SEQ ID No. 1 de la CK8 humaine.

4. Anticorps ou fragment d'anticorps selon l'une des revendications précédentes, se liant uniquement à un fragment d'au moins 10 acides aminés du polypeptide ayant la séquence des acides aminés de la position 338 à la position 367 de la SEQ ID No. 1 de la CK8 humaine.

5. Anticorps ou fragment d'anticorps selon l'une des revendications précédentes pour utilisation pour la prévention ou le traitement des cancers colorectaux invasifs.

6. Composition pharmaceutique **caractérisée en ce qu'**elle comprend un anticorps selon l'une des revendications 1-5 et un véhicule pharmaceutique approprié, pour utilisation pour le traitement des cancers colorectaux.

7. Procédé pour identifier un cancer colorectal ayant un phénotype invasif et/ou métastatique comprenant les étapes :
a) Détection du clivage de la partie C-terminale de la CK8 dans un échantillon préalablement prélevé sur un patient,
b) Classifier le cancer colorectal comme invasif et/ou métastatique lorsque la partie C-terminale de la CK8 est clivée.

8. Procédé pour identifier un cancer colorectal ayant un phénotype invasif et/ou métastatique selon la revendication 7 dans lequel la détection du clivage de la partie C-terminale de la CK8 est effectuée sur un échantillon de cellules de cancer colorectal préalablement prélevé sur un patient.

9. Procédé pour identifier un cancer colorectal ayant un phénotype invasif et/ou métastatique selon la revendication 8 comprenant la détection de la perte totale ou partielle de la partie C-terminale de la CK8 humaine sur des cellules de cancer colorectal avec un anticorps se liant uniquement au polypeptide ayant la séquence des acides aminés de la position 402 à la position 483 de la SEQ ID No. 1 de la CK8 humaine.

10. Procédé pour identifier un cancer colorectal ayant un phénotype invasif et/ou métastatique selon la revendication 9 comprenant en outre la détection de la partie N-terminale de la CK8 humaine sur des cellules tumorales de cancer colorectal avec un anticorps se liant uniquement au polypeptide ayant la séquence des acides aminés de la position 1 à la position 401 de la SEQ ID No. 1 de la CK8 humaine.

11. Procédé pour identifier un cancer colorectal ayant un phénotype invasif et/ou métastatique selon l'une des revendications 7-10 dans lequel la détection du clivage de la partie C-terminale de la CK8 dans un échantillon préalablement prélevé sur un patient est effectuée par immuno-histochimie ou immunofluorescence.

12. Procédé pour identifier un cancer colorectal ayant un phénotype invasif et/ou métastatique selon la revendication 7 dans lequel la détection du clivage de la partie C-terminale de la CK8 est effectuée dans un échantillon de sang préalablement prélevé sur un patient et dans lequel on détecte dans ledit échantillon de sang la partie C-terminale de la CK8 humaine à l'aide d'un anticorps se liant uniquement au polypeptide ayant la séquence des acides aminés de la position 402 à la position 483 de la SEQ ID No. 1 de la CK8 humaine.

13. Kit de diagnostic pour identifier un cancer colorectal ayant un phénotype invasif et/ou métastatique **caractérisé en ce qu'**il comprend un anticorps se liant uniquement au polypeptide ayant la séquence des acides aminés de la position 402 à la position 483 de la SEQ ID No. 1 de la CK8 humaine et un anticorps se liant uniquement au polypeptide ayant la séquence des acides aminés de la position 1 à la position 401 de la SEQ ID No. 1 de la CK8 humaine.

## Patentansprüche

1. Antikörper oder Antikörperfragment, der/das sich nur an das Polypeptid bindet, das die Aminosäurensequenz von der Position 1 bis zur Position 401 der SEQ ID No. 1 der menschlichen CK8 hat, für eine Verwendung zur Behandlung von Kolorektalkarzinomen.

2. Antikörper oder Antikörperfragment nach Anspruch 1, der/das sich speziell an das Polypeptid bindet, das die Aminosäurensequenz von der Position 275 bis zur Position 401 der SEQ ID No. 1 der menschlichen CK8 hat.

3. Antikörper oder Antikörperfragment nach einem der vorangehenden Ansprüche, der/das sich nur an das Polypeptid bildet, das die Aminosäurensequenz von der Position 338 bis zur Position 367 der SEQ ID No. 1 der menschlichen CK8 hat.

4. Antikörper oder Antikörperfragment nach einem der vorangehenden Ansprüche, der/das sich nur an ein Fragment von mindestens 10 Aminosäuren des Polypeptids bindet, das die Aminosäurensequenz von der Position 338 bis zur Position 367 der SEQ ID No. 1 der menschlichen CK8 hat.

5. Antikörper oder Antikörperfragment nach einem der vorangehenden Ansprüche für eine Verwendung zur Vorbeugung oder Behandlung invasiver Kolorektalkarzinome.

6. Pharmazeutische Zusammensetzung, **dadurch gekennzeichnet, dass** sie einen Antikörper nach einem der Ansprüche 1-5 und ein pharmazeutisch geeignetes Vehikel für eine Verwendung zur Behandlung von Kolorektalkarzinomen umfasst.

7. Verfahren zur Identifizierung eines Kolorektalkarzinoms mit einem invasiven und/oder metastasierenden Phänotyp, das die Schritte umfasst:
a) Detektion der Abtrennung des C-terminalen Teils der CK8 in einer zuvor einem Patienten entnommenen Probe,
b) Klassifizieren des Kolorektalkarzinoms als invasiv und/oder metastasierend, wenn der C-terminale Teil der CK8 abgetrennt ist.

8. Verfahren zur Identifizierung eines Kolorektalkarzinoms mit einem invasiven und/oder metastasierenden Phänotyp nach Anspruch 7, wobei die Detektion der Abtrennung des C-terminalen Teils der CK8 bei einer Zellprobe des Kolorektalkarzinoms durchgeführt wird, die zuvor einem Patienten entnommen wurde.

9. Verfahren zur Identifizierung eines Kolorektalkarzinoms mit einem invasiven und/oder metastasierenden Phänotyp nach Anspruch 8, das die Detektion des vollständigen oder teilweisen Verlusts des C-terminalen Teils der menschlichen CK8 in den Zellen des Kolorektalkarzinoms mit einem Antikörper umfasst, der sich nur an das Polypeptid bindet, das die Aminosäurensequenz von der Position 402 bis zur Position 483 der SEQ ID No. 1 der menschlichen CK8 hat.

10. Verfahren zur Identifizierung eines Kolorektalkarzinoms mit einem invasiven und/oder metastasierenden Phänotyp nach Anspruch 9, das ferner die Detektion des N-terminalen Teils der menschlichen CK8 in Tumorzellen des Kolorektalkarzinoms mit einem Antikörper umfasst, der sich nur an das Polypeptid bindet, das die Aminosäurensequenz von der Position 1 bis zur Position 401 der SEQ ID No. 1 der menschlichen CK8 hat.

11. Verfahren zur Identifizierung eines Kolorektalkarzinoms mit einem invasiven und/oder metastasierenden Phänotyp nach einem der Ansprüche 7-10, wobei die Detektion der Abtrennung des C-terminalen Teils der CK8 in einer zuvor einem Patienten entnommenen Probe durch Immunhistochemie oder Immunfluoreszenz durchgeführt wird.

12. Verfahren zur Identifizierung eines Kolorektalkarzinoms mit einem invasiven und/oder metastasierenden Phänotyp nach Anspruch 7, wobei die Detektion der Abtrennung des C-terminalen Teils der CK8 in einer zuvor einem Patienten entnommenen Blutprobe durchgeführt wird, und wobei in der Blutprobe der C-terminale Teil der menschlichen CK8 mit Hilfe eines Antikörpers detektiert wird, der sich nur an das Polypeptid bindet, das die Aminosäurensequenz von der Position 402 bis zur Position 483 der SEQ ID No. 1 der menschlichen CK8 hat.

13. Diagnosekit zur Identifizierung eines Kolorektalkarzinoms mit einem invasiven und/oder metastatisierenden Phänotyp, **dadurch gekennzeichnet, dass** es einen Antikörper umfasst, der sich nur an das Polypeptid bindet, das die Aminosäurensequenz von der Position 402 bis zur Position 483 der SEQ ID No. 1 der menschlichen CK8 hat und einen Antikörper, der sich nur an das Polypeptid bindet, das die Aminosäurensequenz von der Position 1 bis zur Position 401 der SEQ ID No. 1 der menschlichen CK8 hat.

## Claims

1. An antibody or antibody fragment, binding only to the polypeptide having the sequence of the amino acids from position 1 to position 401 of SEQ ID No.1 of human CK8 for use for treating colorectal cancers.

2. The antibody or antibody fragment according to claim 1, binding only to the polypeptide having the sequence of the amino acids from position 275 to position 401 of SEQ ID No.1 of human CK8.

3. The antibody or antibody fragment according to one of the preceding claims, binding only to the polypeptide having the sequence of the amino acids from position 338 to position 367 of SEQ ID No.1 of human CK8.

4. The antibody or antibody fragment according to one of the preceding claims, binding only to a fragment of at least 10 amino acids of the polypeptide having the sequence of the amino acids from position 338 to position 367 of human CK8 SEQ ID No.1.

5. The antibody or antibody fragment according to one of the preceding claims for use for preventing or treating invasive colorectal cancers.

6. A pharmaceutical composition **characterized in that** it comprises an antibody according to one of claims 1-5 and an appropriate pharmaceutical carrier, for use in the treatment of colorectal cancers.

7. A method for identifying a colorectal cancer having an invasive and/or metastatic phenotype comprising the steps of:
a) Detecting the cleavage of the C-terminal part of CK8 in a sample previously taken from a patient,
b) Classifying the colorectal cancer as invasive and/or metastatic when the C-terminal part of CK8 is cleaved.

8. The method for identifying a colorectal cancer having an invasive and/or metastatic phenotype according to claim 7 wherein the detection of the cleavage of the C-terminal part of CK8 is carried out on a sample of colorectal cancer cells previously taken from a patient.

9. The method for identifying a colorectal cancer having an invasive and/or metastatic phenotype according toclaim 8 comprising detecting the total or partial loss of the C-terminal part of human CK8 on colorectal cancer cells with an antibody binding only to the polypeptide having the sequence of the amino acids from position 402 to position 483 of SEQ ID No.1 of human CK8.

10. The method for identifying a colorectal cancer having an invasive and/or metastatic phenotype according to claim 9 further comprising detecting the N-terminal part of human CK8 on colorectal cancer cells with an antibody binding only to the polypeptide having the sequence of the amino acids from position 1 to position 401 of SEQ ID No.1 of human CK8.

11. The method for identifying a colorectal cancer having an invasive and/or metastatic phenotype according to one of claims 7-10 wherein the detection of the cleavage of the C-terminal part of CK8 in a sample previously taken from a patient is carried out by immunohistochemistry or immunofluorescence.

12. The method for identifying a colorectal cancer having an invasive and/or metastatic phenotype according to claim 7 wherein the detection of the cleavage of the C-terminal part of CK8 is carried out in a blood sample previously taken from a patient and wherein the C-terminal part of human CK8 is detected in said blood sample by means of an antibody binding only to the polypeptide having the sequence of the amino acids from position 402 to position 483 of SEQ ID No.1 of human CK8.

13. A diagnostic kit for identifying colorectal cancer having an invasive and/or metastatic phenotype **characterized in that** it comprises an antibody binding only to the polypeptide having the sequence of the amino acids from position 402 to position 483 of SEQ ID No.1 of human CK8 and an antibody binding only to the polypeptide having the sequence of the amino acids from position 1 to position 401 of SEQ ID No.1 of human CK8.
